(19) Europäisches Patentamt
European Patent Office
Office européen des brevets

(11) **EP 4 421 758 A1**

(12) **DEMANDE DE BREVET EUROPEEN**

(43) Date de publication:
**28.08.2024 Bulletin 2024/35**

(21) Numéro de dépôt: **23305233.1**

(22) Date de dépôt: **22.02.2023**

(51) Classification Internationale des Brevets (IPC):
*G06V 30/422* *(2022.01)*

(52) Classification Coopérative des Brevets (CPC):
**G06V 30/422**

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**KH MA MD TN**

(71) Demandeurs:
• **ATOS FRANCE**
**95870 Bezons (FR)**
• **Institut National de la Recherche pour
l'Agriculture, l'Alimentation et l'Environnement
75007 Paris (FR)**

(72) Inventeurs:
• **CHEMCHEM, Amine**
**34070 Montpellier (FR)**

• **MOUMNI, Rida**
**34080 Montpellier (FR)**
• **PIMENTA, Paulo**
**34730 Prades-le-Lez (FR)**
• **REHHALI, El Bachir**
**34090 Montpellier (FR)**
• **SOW, Lalla Aïcha**
**34000 Montpellier (FR)**
• **LAGACHERIE, Philippe**
**34230 Vendémian (FR)**
• **STYC, Quentin**
**37550 Saint-Avertin (FR)**

(74) Mandataire: **Novagraaf Technologies
2 rue Sarah Bernhardt
CS90017
92665 Asnières-sur-Seine Cedex (FR)**

(54) **PROCEDE DE DETERMINATION DE DONNEES PEDOLOGIQUES, PROGRAMME D'ORDINATEUR ET DISPOSITIF ASSOCIE**

(57) L'invention concerne un procédé de détermination d'au moins une caractéristique du sol dans une zone géographique, le procédé étant mis en oeuvre par ordinateur et comprenant les étapes :
- détection, dans au moins une image géoréférencée (8) associée à la zone géographique, d'au moins un symbole (10) représentatif d'un point de mesure de données pédologiques, et d'un identifiant unique associé ;
- pour chaque symbole (10) détecté :
• calcul des coordonnées géographiques du point de mesure correspondant, à partir de la position dudit symbole (10) dans l'image et du géoréférencement de l'image ;
• à partir de l'identifiant unique correspondant, identification, dans une base de données pédologiques associées à la zone géographique, des données pédologiques correspondant au point de mesure respectif.

Figure 2

## Description

La présente invention concerne un procédé de détermination d'au moins une caractéristique du sol dans une zone géographique.

L'invention concerne également un programme d'ordinateur et un dispositif mettant en oeuvre un tel procédé.

L'invention s'applique au domaine de la pédologie, et plus particulièrement à la détermination des données pédologiques dans une zone géographique.

## État de la technique

Pour optimiser l'exploitation des sols dans une zone géographique donnée (par exemple pour sélectionner la culture permettant une récolte optimale, ou encore pour gérer au mieux les réserves d'eau disponibles), il convient de connaître les caractéristiques du sol en chaque point de cette zone.

De façon classique, pour connaître les caractéristiques du sol en un point donné, il est connu de réaliser deux analyses complémentaires audit point.

Plus précisément, la première analyse consiste à réaliser un sondage du sol en prélevant des carottes de terre à intervalles de profondeur réguliers (par exemple pour effectuer des analyses chimiques et physiques). De tels sondages sont facilement réalisables en pratique.

En outre, la deuxième analyse consiste à réaliser une coupe verticale du sol (appelée « fosse pédologique ») pour observer le profil du sol, c'est-à-dire les différentes strates composant le sol, de la surface jusqu'à la roche sous-jacente. De telles fosses pédologiques sont creusées sur une profondeur pouvant aller jusqu'à plus de deux mètres, ce qui rend irréaliste leur mise en oeuvre systématique.

Toutefois, les caractéristiques du sol évoluant peu ou de manière prévisible avec le temps, les données relatives à des campagnes pédologiques anciennes peuvent être utilisées pour estimer les caractéristiques actuelles du sol.

Néanmoins, l'utilisation des données pédologiques relatives à des campagnes anciennes n'est pas satisfaisant.

En effet, de telles données sont souvent uniquement disponibles sur des documents papier, généralement de mauvaise qualité et dans un format qui diffère d'un document à l'autre. Par conséquent, leur traitement est effectué manuellement, ce qui représente un travail fastidieux et coûteux en temps.

En outre, ces données ne sont généralement pas géoréférencées, ce qui rend difficile l'obtention d'une estimation localement précise d'une propriété du sol calculée à partir de ces données.

Un but de la présente invention est de remédier à au moins un des inconvénients de l'état de la technique.

Un autre but de l'invention est de proposer une solution qui soit automatique, rapide et fiable pour évaluer les caractéristiques pédologiques en tout point d'une zone géographique.

## Exposé de l'invention

À cet effet, l'invention a pour objet un procédé du type précité, le procédé étant mis en oeuvre par ordinateur et comprenant les étapes :

- détection, dans au moins une image géoréférencée associée à la zone géographique, d'au moins un symbole représentatif d'un point de mesure de données pédologiques, et d'un identifiant unique associé ;
- pour chaque symbole détecté :

  • calcul des coordonnées géographiques du point de mesure correspondant, à partir de la position dudit symbole dans l'image et du géoréférencement de l'image ;
  • à partir de l'identifiant unique correspondant, identification, dans une base de données pédologiques associées à la zone géographique, des données pédologiques correspondant au point de mesure respectif.

En effet, grâce à un tel procédé, un lien univoque est établi de façon automatique entre les points de mesure, les données pédologiques correspondantes et leur géolocalisation respective. Ceci permet de surmonter le problème selon lequel les données pédologiques des campagnes de mesure anciennes n'étaient pas géoréférencées.

Ce lien est établi de façon automatique, rapide et fiable, sans nécessiter l'intervention d'un opérateur humain.

Ceci est particulièrement avantageux, dans la mesure où la connaissance des coordonnées géographiques exactes de chaque point de mesure, ainsi que le stockage, sous forme numérique, des données pédologiques correspondantes dans la base de données, autorise une simulation de nouvelles données pédologiques, cette fois-ci en tout point de la zone géographique, et ce de façon totalement automatisée.

De façon avantageuse, le procédé selon l'invention présente une ou plusieurs des caractéristiques suivantes, prises isolément ou selon toute combinaison techniquement possible :

le procédé comprend, en outre, une estimation, à partir des coordonnées géographiques calculées pour chaque point de mesure et des données pédologiques identifiées correspondantes, des données pédologiques en tout point de la zone géographique ;

l'estimation des données pédologiques en tout point de la zone géographique comprend la mise en oeuvre d'au moins un modèle d'intelligence artificielle, de préférence une régression quantile de forêt rapide ;

pour chaque point de la zone géographique,

l'estimation des données pédologiques comporte, en outre, une évaluation d'une incertitude d'estimation correspondante ;

l'évaluation de l'incertitude d'estimation comprend :

- calcul d'une incertitude en chaque point de mesure, à partir d'une erreur du modèle entre les données pédologiques estimées au point de mesure et les données pédologiques associées audit point de mesure dans la base de données pédologiques ;
- propagation de l'incertitude calculée en chaque point de la zone géographique ;

l'estimation des données pédologiques est précédée d'une interpolation des profondeurs entre points de mesure ;

l'interpolation des profondeurs comprend la mise en oeuvre de splines à conservation de masse ;

l'estimation des données pédologiques en tout point de la zone géographique est également fonction de covariables de sol de la zone géographique ;

les covariables comportent des informations relatives à la topographie, des informations relatives à la géologie et/ou des informations relatives à l'occupation des sols de la zone géographique ;

l'au moins une caractéristique du sol comprend un réservoir utile en eau ;

les données pédologiques comprennent des profondeurs supérieure et inférieure, des pourcentages massiques de graviers et de cailloux, un pourcentage pondéral de terre fine, une densité apparente, une humidité équivalente et/ou un coefficient structural pour chaque strate ;

le procédé comprend, préalablement à l'étape d'identification, une étape de population de la base de données pédologiques comportant :

- détection, dans chaque image représentative d'une fiche de sondage réalisé en un point de mesure correspondant de la zone géographique, d'un identifiant du point de mesure et d'un identifiant de la zone géographique ;
- extraction, depuis l'image, des données pédologiques associées ;
- génération de l'identifiant unique à partir de l'identifiant du point de mesure et de l'identifiant de la zone géographique détectés ; et
- enregistrement, dans la base de données pédologiques, des données pédologiques extraites en relation avec l'identifiant unique du point de mesure ;

l'extraction des données pédologiques comporte un traitement comprenant : une suppression des lignes vides ou partiellement remplies, une correction de discontinuités de profondeur du sol, une correction de profondeurs erronées, une correction de décalages de lignes et/ou une suppression de doublons.

Selon un autre aspect de l'invention, il est proposé un programme d'ordinateur comprenant des instructions exécutables qui, lorsqu'elles sont exécutées par ordinateur, mettent en en oeuvre les étapes du procédé tel que défini ci-dessus.

Le programme d'ordinateur peut être en tout langage informatique, tel que par exemple en langage machine, en C, C++, JAVA, Python, etc.

Selon un autre aspect de l'invention, il est proposé un dispositif pour la détermination d'au moins une caractéristique du sol dans une zone géographique, le dispositif comprenant une base de données et une unité de traitement,

la base de données étant configurée pour stocker des données pédologiques associées à la zone géographique,

l'unité de traitement étant configurée pour :

- détecter, dans au moins une image géoréférencée associée à la zone géographique, au moins un symbole représentatif d'un point de mesure de données pédologiques, et d'un identifiant unique associé ;
- pour chaque symbole détecté :

  • calculer des coordonnées géographiques du point de mesure correspondant, à partir de la position dudit symbole dans l'image et du géoréférencement de l'image ;
  • à partir de l'identifiant unique correspondant, identifier, dans la base de données, des données pédologiques correspondant au point de mesure respectif.

Le dispositif selon l'invention peut être tout type d'appareil tel qu'un serveur, un ordinateur, une tablette, un calculateur, un processeur, une puce informatique, programmé pour mettre en oeuvre le procédé selon l'invention, par exemple en exécutant le programme d'ordinateur selon l'invention.

## Brève description des figures

L'invention sera mieux comprise à la lecture de la description qui va suivre, donnée uniquement à titre d'exemple non limitatif, et faite en se référant aux dessins annexés sur lesquelles :

- la figure 1 est une représentation schématique d'un dispositif selon l'invention ; et

- la figure 2 est un exemple d'image géoréférencée fournie en entrée du dispositif de la figure 1.

## Description des figures et modes de réalisation

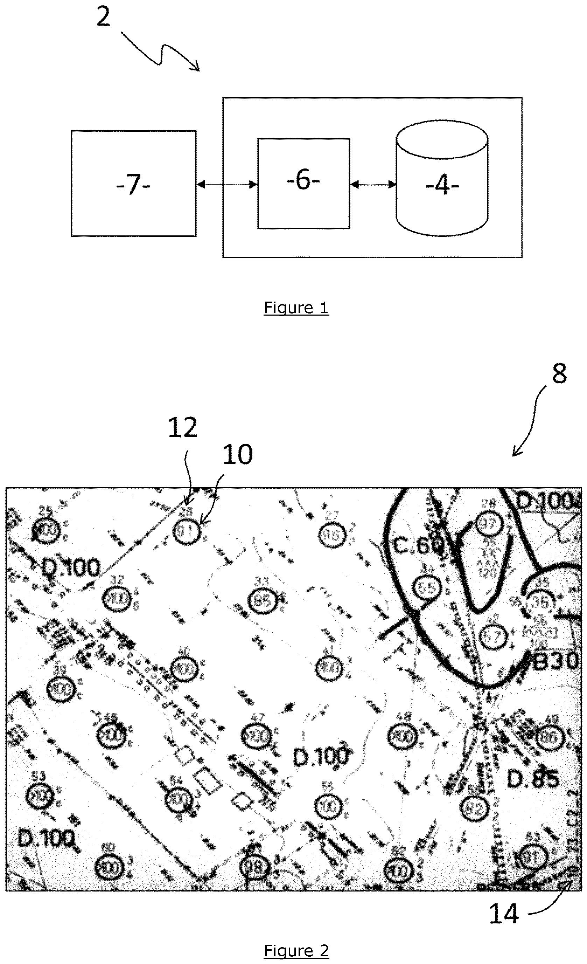

Un dispositif 2 selon l'invention est schématiquement illustré par la figure 1.

Le dispositif 2 est destiné à déterminer au moins une caractéristique du sol dans une ou plusieurs zone(s) géographique(s) donnée(s).

Par exemple, une telle caractéristique est un réservoir utile en eau.

Le dispositif 2 comporte une base de données 4 (encore appelée « mémoire ») et une unité de traitement 6 reliées entre elles. De préférence, le dispositif 2 comporte également un afficheur 7 connecté à l'unité de traitement 6.

Le dispositif 2 est susceptible de se présenter sous une forme matérielle, telle qu'un ordinateur, un serveur, un processeur, une puce électronique, etc. Alternativement, ou de façon additionnelle, le dispositif 2 est susceptible de se présenter sous une forme logicielle telle qu'un programme d'ordinateur, ou une application, par exemple une application pour un appareil utilisateur de type tablette ou smartphone.

**Mémoire 4**

La mémoire 4 est configurée pour stocker des données pédologiques associées à chaque zone géographique.

Plus précisément, la mémoire 4 est configurée pour stocker des données pédologiques associées à au moins un point de mesure de la zone géographique.

Par « point de mesure », il est entendu, au sens de la présente invention, un point de la zone géographique où des données pédologiques ont été collectées, en particulier par la mise en oeuvre d'une fosse pédologique ou d'un sondage pédologique (prélèvement de carottes de terre à des profondeurs successives).

Par exemple, de telles données pédologiques comprennent, pour chaque strate du sol (encore appelée « horizon pédologique »), des profondeurs supérieure et inférieure, des pourcentages massiques de graviers et de cailloux, un pourcentage pondéral de terre fine, une densité apparente, une humidité équivalente et/ou un coefficient structural.

Dans la mémoire 4, chaque point de mesure est identifié grâce à un identifiant unique correspondant. En outre, chaque identifiant unique est mis en correspondance, dans la mémoire 4, avec les données pédologiques relevées au point de mesure associé.

De préférence, la mémoire 4 est également configurée pour stocker des covariables de sol relatives à la zone géographique.

De façon classique, de telles covariables comportent des informations relatives à la topographie, des informations relatives à la géologie et/ou des informations relatives à l'occupation des sols de la zone géographique.

**Unité de traitement 6**

L'unité de traitement 6 est une unité de traitement matérielle, telle qu'un processeur, une puce électronique, un calculateur, un ordinateur, un serveur, etc. Alternativement, ou de façon additionnelle, l'unité de traitement 6 est une unité de traitement logicielle, telle qu'une application, un programme d'ordinateur, une machine virtuelle, etc.

L'unité de traitement 6 est destinée à localiser les points de mesures et à identifier les données pédologiques correspondantes.

Avantageusement, l'unité de traitement 6 est également configurée pour estimer des données pédologiques en tout point de la zone géographique à partir des données pédologiques aux points de mesure. Ceci est avantageux, dans la mesure où la connaissance en tout point des données pédologiques autorise la détermination, en tout point de la zone géographique, des caractéristiques du sol.

De préférence, l'unité de traitement 6 est également configurée pour, au préalable, peupler la base de données 4, c'est-à-dire enregistrer, dans la base de données 4, les données pédologiques relevées aux points de mesure.

*Peuplement de la base de données*

Pour peupler la mémoire 4, l'unité de traitement 6 est configurée pour recevoir au moins une image représentative de données pédologiques relevées en un point de mesure de la zone géographique.

Par exemple, une telle image résulte de la numérisation d'une fiche de sondage relative à un sondage pédologique réalisé en un point de mesure donné.

Alternativement, ou de façon complémentaire, une telle image résulte de la numérisation d'un profil de sol relatif à une fosse pédologique réalisée en un point de mesure donné.

L'unité de traitement 6 est également configurée pour détecter, dans chaque image reçue, un identifiant du point de mesure et un identifiant de la zone géographique. En particulier, pour réaliser une telle détection, l'unité de traitement 6 est configurée pour mettre un oeuvre un logiciel de reconnaissance de caractères, par exemple configuré pour implémenter un modèle d'intelligence artificielle.

Avantageusement, une telle détection se fonde, au moins en partie, sur une connaissance préalable des images. Par exemple, l'unité de traitement 6 est paramétrée pour rechercher les identifiants du point de mesure et de la zone géographique dans une partie spécifique de l'image dont on sait qu'elle est susceptible de contenir lesdits identifiants. Ceci est avantageux, dans la mesure où un tel paramétrage réduit le temps de traitement de l'image et en accroît la fiabilité.

L'unité de traitement 6 est également configurée pour extraire, les données pédologiques de l'image. En particulier, pour réaliser une telle extraction, l'unité de traitement 6 est configurée pour mettre un oeuvre un logiciel de reconnaissance de caractères, par exemple configuré pour implémenter un modèle d'intelligence artificielle.

Avantageusement, une telle détection se fonde, au moins en partie, sur une connaissance préalable des images. Par exemple, l'unité de traitement 6 est paramétrée pour rechercher chacune des données pédologiques dans une partie spécifique de l'image dont on sait qu'elle est susceptible de contenir lesdites données. Ceci est avantageux, dans la mesure où un tel paramétrage réduit le temps de traitement de l'image et en accroît la fiabilité.

De façon avantageuse, l'unité de traitement 6 est, en outre, configurée pour mettre en oeuvre, lors d'une telle extraction, un traitement des données extraites. Un tel traitement comprend une suppression des lignes vides ou partiellement remplies, une correction de discontinuités de profondeur du sol, une correction de profondeurs erronées, une correction de décalages de lignes et/ou une suppression de doublons. Cette opération est avantageuse, dans la mesure où elle conduit à une harmonisation des données extraites et/ou à la suppression de données incorrectes, ce qui résulte en une réduction du nombre de données extraites erronées sur lesquelles l'estimation des données pédologiques dans la zone géographique serait susceptible de se fonder.

L'unité de traitement 6 est également configurée pour générer, pour chaque point de mesure, l'identifiant unique mentionné précédemment. Plus précisément, pour chaque point de mesure, l'unité de traitement 6 est configurée pour générer l'identifiant unique à partir de l'identifiant du point de mesure et de l'identifiant de la zone géographique détectés. Par exemple, l'identifiant unique est le résultat de la concaténation de l'identifiant du point de mesure et de l'identifiant de la zone géographique.

En outre, pour chaque point de mesure, l'unité de traitement 6 est configurée pour enregistrer, dans la mémoire 4, les données pédologiques extraites correspondantes en relation avec l'identifiant unique respectif.

À l'issue de ce processus, la base de données 4 stocke, pour chaque point de mesure, les données pédologiques correspondantes, en relation avec l'identifiant unique respectif.

*Détermination des coordonnées géographiques de chaque point de mesure*

L'unité de traitement 6 est également configurée pour faire correspondre les données pédologiques stockées dans la mémoire 4 à des coordonnées géographiques. Plus précisément, l'unité de traitement 6 est configurée pour réaliser une telle correspondance sur la base d'au moins une image géoréférencée associée à la zone géographique et desdites données pédologiques stockées dans la mémoire 4. Dans ce cas, chaque image géoréférencée forme, par exemple, le plus petit carré géoréférencé (encore appelé « mapillon ») d'un carroyage d'une étendue dans laquelle des données pédologiques ont été collectées.

Une telle image géoréférencée est une image associée à la zone géographique (par exemple une image représentative d'au moins une partie de la zone géographique) et dans laquelle des repères (également appelés « symboles ») associés aux points de mesure ont été placés.

Par « image géoréférencée », il est entendu, au sens de la présente demande, une image pour laquelle au moins trois points présentent des coordonnées géographiques connues.

Un exemple d'une telle image géoréférencée 8 est visible sur la figure 2.

Comme cela apparaît sur la figure 2, chaque symbole 10 (ici, un cercle) est disposé, sur l'image géoréférencée 8, à une position qui correspond à la position réelle du point de mesure correspondant dans la zone géographique.

En outre, chaque symbole 10 est associé à un identifiant 12 du point de mesure. Sur l'image géoréférencée 8, l'identifiant 12 du point de mesure prend la forme d'un nombre à deux chiffres situé au-dessus du symbole 10 correspondant.

L'image géoréférencée 8 comprend, en outre, un identifiant 14 de la zone géographique. Sur l'image géoréférencée 8, l'identifiant 14 de la zone géographique représentée, situé dans le coin inférieur droit, est : 10_23_C2_2.

Dans ce cas, l'unité de traitement 6 est configurée pour détecter, dans chaque image géoréférencée, chaque symbole 10 représentatif d'un point de mesure. Par exemple, pour réaliser une telle détection, l'unité de traitement 6 est configurée pour mettre en oeuvre un algorithme de reconnaissance de forme.

En outre, pour chaque symbole 10, l'unité de traitement 6 est configurée pour détecter l'identifiant 12 correspondant. Par exemple, l'unité de traitement 6 est paramétrée pour rechercher l'identifiant 12 dans une partie de l'image géoréférencée 8 choisie relativement à la position du symbole 10 dans ladite image 8, et dont on sait qu'elle est susceptible de contenir l'identifiant 12.

L'unité de traitement 6 est également configurée pour détecter l'identifiant 14 de la zone géographique. Par exemple, l'unité de traitement 6 est paramétrée pour rechercher l'identifiant 14 dans une partie de l'image géoréférencée 8 dont on sait qu'elle est susceptible de contenir l'identifiant 14.

De préférence, pour détecter les identifiants 12, 14, l'unité de traitement 6 est configurée pour mettre en oeuvre un logiciel de reconnaissance de caractères.

En outre, pour chaque point de mesure associé à un symbole 10 détecté, l'unité de traitement 6 est configurée pour déterminer l'identifiant unique à partir de l'identifiant 12 du point de mesure et de l'identifiant 14 de la zone géographique.

Pour chaque symbole 10 détecté, l'unité de traitement 6 est également configurée pour calculer les coordonnées géographiques du point de mesure correspondant, à partir de la position du symbole 10 dans l'image géoréférencée 8 et du géoréférencement de ladite image 8. Par exemple, pour chaque symbole 10 détecté, l'unité de traitement 6 est configurée pour calculer les coordonnées géographiques du point de mesure correspondant à partir de la position du barycentre du symbole 10 dans l'image géoréférencée 8.

En outre, pour chaque point de mesure, l'unité de traitement 6 est configurée pour identifier, dans la mémoire 4, des données pédologiques correspondantes.

Plus précisément, l'unité de traitement 6 est configurée pour déterminer l'identifiant unique à partir de l'identifiant 12 du point de mesure et de l'identifiant 14 de la zone géographique.

L'unité de traitement 6 est configurée pour ensuite charger, depuis la mémoire 4, et pour chaque point de mesure, les données pédologiques associées à l'identifiant unique déterminé qui lui est associé.

De préférence, l'unité de traitement 6 est également configurée pour enregistrer, depuis la mémoire 4, et pour chaque point de mesure, les coordonnées géographiques déterminées correspondantes.

*Calcul des données pédologiques dans la zone géographique*

De préférence, l'unité de traitement 6 est également configurée pour estimer des données pédologiques en tout point de la zone géographique, et ce à partir des coordonnées géographiques calculées pour chaque point de mesure et des données pédologiques identifiées correspondantes.

Par exemple, pour réaliser une telle estimation, l'unité de traitement 6 est configurée pour mettre en oeuvre un modèle d'intelligence artificielle préalablement entraîné, de préférence une régression quantile de forêt rapide. De préférence encore, l'unité de traitement 6 est configurée pour réaliser une telle estimation en mettant en oeuvre, pour chaque horizon pédologique, un modèle d'intelligence artificielle respectif.

Avantageusement, l'unité de traitement 6 est configurée pour réaliser cette estimation sur la base des covariables de sol de la zone géographique stockées dans la mémoire 4. Par exemple, dans le cas du recours à un modèle d'intelligence artificielle pour estimer les données pédologiques, le modèle est également paramétré pour prendre en entrée lesdites covariables de sol afin de réaliser ladite estimation.

De préférence, pour réaliser une telle estimation, l'unité de traitement 6 est configuré pour d'abord mettre en oeuvre une interpolation des profondeurs entre les points de mesure. De cette façon, une harmonisation en profondeur entre les données pédologiques associées aux différents points de mesure est réalisée.

Par exemple, l'unité de traitement 6 est configurée pour réaliser une telle interpolation à plusieurs profondeurs, telles que 30 cm, 60 cm, 100 cm et 200 cm.

De cette façon, une pluralité d'horizons pédologiques est définie, chacun étant compris entre deux profondeurs successives.

En particulier, l'unité de traitement 6 est configurée pour mettre en oeuvre des splines à conservation de masse afin de réaliser une telle interpolation. En variante, pour réaliser une telle interpolation, l'unité de traitement 6 est configurée pour mettre en oeuvre toute autre méthode d'interpolation adaptée, telle qu'une pondération inverse à la distance (ou « *inverse distance weighting* » en anglais), ou encore un krigeage (ou « *kriging* » en anglais).

De façon avantageuse, pour chaque point de la zone géographique, outre les données pédologiques correspondantes, l'unité de traitement 6 est également configurée pour évaluer une incertitude d'estimation correspondante.

De préférence encore, pour chaque point de la zone géographique, une telle évaluation d'incertitude est réalisée pour chaque horizon pédologique.

Par exemple, dans le cas où un modèle d'intelligence artificielle est entraîné pour estimer les données pédologiques en tout point de la zone géographique, une validation croisée (dont l'implémentation est connue de l'homme du métier) est mise en oeuvre lors de l'entraînement du modèle sur la base des données pédologiques (éventuellement après interpolation) associées aux points de mesure. Ceci amène à déterminer une incertitude (c'est-à-dire une fiabilité) de l'estimation des données pédologiques faite par le modèle, à chaque point de mesure. De préférence, et comme indiqué précédemment, une telle incertitude est évaluée pour chaque horizon pédologique.

Alternativement, l'unité de traitement 6 est configurée pour mettre en oeuvre toute autre méthode de calcul d'incertitude sur l'estimation à chaque point de mesure, qui soit fonction d'une erreur du modèle entre, d'une part, les données pédologiques estimées au point de mesure et, d'autre part, les données pédologiques effectivement mesurées (et donc associées audit point de mesure dans la mémoire 4).

En particulier, pour effectuer une telle évaluation d'incertitude, l'unité de traitement 6 est configurée pour propager, en chaque point de la zone géographique, l'incertitude calculée aux points de mesure. De préférence, une telle propagation est réalisée de façon distincte pour chaque horizon pédologique.

L'unité de traitement 6 est également configurée pour calculer au moins une caractéristique du sol en tout point de la zone géographique à partir des données pédologiques estimées. De cette façon, pour chaque zone géographique, une carte des variations spatiales de chaque caractéristique du sol est obtenue, ainsi que, de préférence, une carte des incertitudes associées.

Par exemple, l'unité de traitement 6 est configurée pour calculer un réservoir utile en eau des sols, par exemple en mettant en oeuvre la formule :

$$RU = \sum_{i} dh_i\, bd_i \left(\frac{100\text{-}st_i}{100}\right) b_i\, H_{eqi}$$

où RU est le réservoir utile en eau en un point donné de la zone géographique,

$H_{eqi}$ est l'humidité équivalente de l'horizon pédologique i audit point,

$b_i$ est le coefficient de texture de l'horizon pédologique i audit point,

$st_i$ est la teneur volumique en éléments grossiers (c'est-à-dire présentant un diamètre supérieur à 2 cm) de l'horizon pédologique i audit point,

$bd_i$ est la densité apparente de la terre fine de l'horizon pédologique i audit point, et

$dh_i$ est l'épaisseur de l'horizon pédologique i audit point.

De préférence, pour chaque zone géographique, l'unité de traitement 6 est également configurée pour transmettre, à destination de l'afficheur 7, la carte correspondante des variations spatiales de chaque caractéristique du sol pour son affichage, et, le cas échéant, la carte des incertitudes associées.

Le recours à une régression quantile de forêt rapide est avantageux, les inventeurs ayant constaté l'effet surprenant selon lequel un tel modèle d'intelligence artificielle présente des performances très satisfaisantes pour la présente application.

L'erreur d'estimation faite par le modèle d'intelligence artificielle aux points de mesure est susceptible d'être aisément obtenue. Par conséquent, le recours à une propagation de ladite erreur d'estimation est avantageuse, dans la mesure où elle autorise une détermination de l'incertitude en tout point de la zone géographique qui est peu gourmande en temps de calcul.

Le recours à une interpolation des profondeurs entre points de mesure est avantageux, dans la mesure où il conduit à une harmonisation des profondeurs des horizons pédologiques, qui sont susceptibles de varier fortement d'un point de mesure à un autre. De cette façon, l'estimation des données pédologiques est réalisée pour de nouveaux horizons fictifs, de profondeur fixe.

La mise en oeuvre de splines à conservation de masse est avantageuse, dans la mesure où les différences entre les données pédologiques avant et après interpolation sont minimes. Ceci résulte du fait que la moyenne des valeurs pour les horizons fictifs générés et la valeur initiale introduite pour les générer sont égales.

Le recours aux covariables de sol pour l'estimation des données pédologiques sur toute la zone géographique est avantageux, dans la mesure où il accroît la fiabilité de l'estimation par la prise en compte de la nature réelle du terrain.

La détermination du réservoir utile en eau est avantageuse, dans la mesure où la connaissance d'une telle caractéristique est utile pour optimiser l'exploitation des sols, par exemple pour sélectionner la culture permettant une récolte satisfaisante, ou encore pour permettre une meilleure gestion des réserves d'eau disponibles.

L'estimation des pourcentages massiques de graviers/cailloux, du pourcentage pondéral de terre fine, de la densité apparente, de l'humidité équivalente et/ou du coefficient structural est avantageuse, dans la mesure où elle autorise une estimation plus fiable du réservoir utile en eau.

**Fonctionnement**

Le fonctionnement du dispositif 2 va maintenant être décrit.

Au cours d'une étape d'initialisation, l'unité de traitement 6 peuple la mémoire 4. Plus précisément, l'unité de traitement 6 reçoit une ou plusieurs image(s) chacune représentative de données pédologiques relevées en un point de mesure de la zone géographique.

Puis, l'unité de traitement 6 détecte, dans chaque image reçue, l'identifiant du point de mesure et l'identifiant de la zone géographique.

L'unité de traitement 6 extrait également les données pédologiques de l'image. Avantageusement, lors de cette extraction, l'unité de traitement 6 met en oeuvre un traitement des données extraites pour réduire le nombre de données erronées.

L'unité de traitement 6 génère également, pour chaque point de mesure, l'identifiant unique mentionné précédemment, en particulier l'identifiant du point de mesure et de l'identifiant de la zone géographique détectés.

Puis, pour chaque point de mesure, l'unité de traitement 6 enregistre, dans la mémoire 4, les données pédologiques extraites correspondantes en relation avec l'identifiant unique respectif.

Puis, au cours d'une étape de localisation, au moins une image géoréférencée est fournie en entrée de l'unité de traitement 6.

Dans ce cas, l'unité de traitement 6 détecte chaque symbole 10 représentatif d'un point de mesure, l'identifiant 12 correspondant, ainsi que l'identifiant 14 de la zone géographique.

Puis, pour chaque symbole 10 détecté, l'unité de traitement 6 calcule les coordonnées géographiques du point de mesure correspondant, et détermine l'identifiant unique du point de mesure correspondant.

Puis, pour chaque point de mesure, l'unité de traitement 6 identifie, dans la mémoire 4, les données pédologiques correspondantes au moyen de l'identifiant unique déterminée, et les charge.

Puis, au cours d'une étape de calcul, l'unité de traitement 6 estime les données pédologiques en tout point de la zone géographique, à partir des coordonnées géographiques calculées pour chaque point de mesure et des données pédologiques identifiées correspondantes.

Avantageusement, pour chaque point de la zone géographique, l'unité de traitement 6 évalue également une incertitude d'estimation correspondante.

Puis, l'unité de traitement 6 calcule chaque caractéristique du sol à partir des données pédologiques estimées.

Puis, de préférence, l'unité de traitement 6 transmet, à destination de l'afficheur 7, la carte des variations spatiales de chaque caractéristique du sol (et, le cas échéant, la carte des incertitudes associées) pour son affichage.

**Revendications**

1. Procédé de détermination d'au moins une caractéristique du sol dans une zone géographique, le procédé étant mis en oeuvre par ordinateur et comprenant les étapes :

   - détection, dans au moins une image géoréférencée (8) associée à la zone géographique, d'au moins un symbole (10) représentatif d'un point de mesure de données pédologiques, et d'un identifiant unique associé ;
   - pour chaque symbole (10) détecté :

     • calcul des coordonnées géographiques du point de mesure correspondant, à partir de la position dudit symbole (10) dans l'image et du géoréférencement de l'image ;
     • à partir de l'identifiant unique correspondant, identification, dans une base de données pédologiques associées à la zone géographique, des données pédologiques correspondant au point de mesure respectif.

2. Procédé selon la revendication 1, comprenant, en outre, une estimation, à partir des coordonnées géographiques calculées pour chaque point de mesure et des données pédologiques identifiées correspondantes, des données pédologiques en tout point de la zone géographique.

3. Procédé selon la revendication 2, dans lequel l'estimation des données pédologiques en tout point de la zone géographique comprend la mise en oeuvre d'au moins un modèle d'intelligence artificielle, de préférence une régression quantile de forêt rapide.

4. Procédé selon la revendication 2 ou 3, dans lequel, pour chaque point de la zone géographique, l'estimation des données pédologiques comporte, en outre, une évaluation d'une incertitude d'estimation correspondante.

5. Procédé selon la revendication 4 lorsqu'elle dépend de la revendication 3, dans lequel l'évaluation de l'incertitude d'estimation comprend :

   - calcul d'une incertitude en chaque point de mesure, à partir d'une erreur du modèle entre les données pédologiques estimées au point de mesure et les données pédologiques associées audit point de mesure dans la base de données pédologiques ;
   - propagation de l'incertitude calculée en chaque point de la zone géographique.

6. Procédé selon l'une quelconque des revendications 2 à 5, dans lequel l'estimation des données pédologiques est précédée d'une interpolation des profondeurs entre points de mesure.

7. Procédé selon la revendication 6, dans lequel l'interpolation des profondeurs comprend la mise en oeuvre de splines à conservation de masse.

8. Procédé selon l'une quelconque des revendications 2 à 7, dans lequel l'estimation des données pédologiques en tout point de la zone géographique est également fonction de covariables de sol de la zone géographique.

9. Procédé selon la revendication 8, dans lequel les covariables comportent des informations relatives à la topographie, des informations relatives à la géologie et/ou des informations relatives à l'occupation des sols de la zone géographique.

10. Procédé selon l'une quelconque des revendications 1 à 9, dans lequel l'au moins une caractéristique du sol comprend un réservoir utile en eau.

11. Procédé selon l'une quelconque des revendications 1 à 10, dans lequel les données pédologiques comprennent des profondeurs supérieure et inférieure, des pourcentages massiques de graviers et de cailloux, un pourcentage pondéral de terre fine, une densité apparente, une humidité équivalente et/ou un coefficient structural pour chaque strate.

12. Procédé selon l'une quelconque des revendications 1 à 11, comprenant, préalablement à l'étape d'identification, une étape de population de la base de données pédologiques comportant :

   - détection, dans chaque image représentative d'une fiche de sondage réalisé en un point de mesure correspondant de la zone géographique, d'un identifiant (12) du point de mesure et d'un identifiant (14) de la zone géographique ;
   - extraction, depuis l'image, des données pédologiques associées ;
   - génération de l'identifiant unique à partir de l'identifiant (12) du point de mesure et de l'identifiant (14) de la zone géographique détectés ; et
- enregistrement, dans la base de données pédologiques, des données pédologiques extraites en relation avec l'identifiant unique du point de mesure.

13. Procédé selon la revendication 12, dans lequel l'extraction des données pédologiques comporte un traitement comprenant : une suppression des lignes vides ou partiellement remplies, une correction de discontinuités de profondeur du sol, une correction de profondeurs erronées, une correction de décalages de lignes et/ou une suppression de doublons.

14. Programme d'ordinateur comprenant des instructions exécutables qui, lorsqu'elles sont exécutées par ordinateur, mettent en en oeuvre les étapes du procédé selon l'une quelconque des revendications 1 à 13.

15. Dispositif pour la détermination d'au moins une caractéristique du sol dans une zone géographique, le dispositif comprenant une base de données et une unité de traitement,

la base de données étant configurée pour stocker des données pédologiques associées à la zone géographique,
l'unité de traitement étant configurée pour :

- détecter, dans au moins une image géoréférencée (8) associée à la zone géographique, au moins un symbole (10) représentatif d'un point de mesure de données pédologiques, et d'un identifiant unique associé ;
- pour chaque symbole (10) détecté :

• calculer des coordonnées géographiques du point de mesure correspondant, à partir de la position dudit symbole (10) dans l'image et du géoréférencement de l'image ;
• à partir de l'identifiant unique correspondant, identifier, dans la base de données, des données pédologiques correspondant au point de mesure respectif.

2

Figure 1

8

12

10

14

Figure 2

**EP 4 421 758 A1**

Europäisches
Patentamt

European
Patent Office

Office européen
des brevets

# RAPPORT DE RECHERCHE EUROPEENNE

Numéro de la demande

**EP 23 30 5233**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | LUYANG LI ET AL: "Integrated text and line-art extraction from a topographic map", INTERNATIONAL JOURNAL ON DOCUMENT ANALYSIS AND RECOGNITION, 30 juin 2000 (2000-06-30), pages 177-185, XP055506057, [extrait le 2018-09-11] * le document en entier * ----- | 1-15 | INV. G06V30/422 |
| A | CN 110 096 565 B (JIANGSU PROVINCE SURVEYING & MAPPING ENGINEERING INST) 29 juin 2021 (2021-06-29) * le document en entier * ----- | 1-15 | |
| A | YAO-YI CHIANG ET AL: "A Survey of Digital Map Processing Techniques", ACM COMPUTING SURVEYS, ACM, NEW YORK, NY, US, US, vol. 47, no. 1, 1 mai 2014 (2014-05-01), pages 1-44, XP058048815, ISSN: 0360-0300, DOI: 10.1145/2557423 * le document en entier * ----- | 1-15 | |
| | | | **DOMAINES TECHNIQUES RECHERCHES (IPC)** |
| | | | G06V |

Le présent rapport a été établi pour toutes les revendications

| Lieu de la recherche | Date d'achèvement de la recherche | Examinateur |
|---|---|---|
| La Haye | 27 juin 2023 | de Bont, Emma |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
................................................................
& : membre de la même famille, document correspondant

EPO FORM 1503 03.82 (P04C02)

11

**ANNEXE AU RAPPORT DE RECHERCHE EUROPEENNE**
**RELATIF A LA DEMANDE DE BREVET EUROPEEN NO.**

**EP 23 30 5233**

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

**27−06−2023**

| Document brevet cité au rapport de recherche | Date de publication | Membre(s) de la famille de brevet(s) | Date de publication |
|---|---|---|---|
| CN 110096565 B | 29−06−2021 | AUCUN | |

Pour tout renseignement concernant cette annexe : voir Journal Officiel de l'Office européen des brevets, No.12/82

EPO FORM P0460